# EUROPEAN PATENT SPECIFICATION

(11) **EP 3 480 695 B1**
(45) Date of publication and mention of the grant of the patent: **18.12.2019**
(21) Application number: 17199832.1
(22) Date of filing: 03.11.2017
(51) Int. Cl.: G06F 9/50

(54) **CLOUD INFRASTRUCTURE RECOMMENDATION SYSTEM**
CLOUD-INFRASTRUKTUR-EMPFEHLUNGSSYSTEM
SYSTÈME DE RECOMMANDATION D'INFRASTRUCTURE EN NUAGE

(43) Date of publication of application: 08.05.2019
(73) Proprietor: FUJITSU LIMITED, Kanagawa 211-8588 (JP)
(72) Inventor: VALIN, Raul, 28003 Madrid (ES)
(74) Representative: Haseltine Lake Kempner LLP

(56) References cited:
- US-A1- 2013 198 723
- US-A1- 2014 068 053
- US-B1- 9 645 628

## Description

The present invention relates to a cloud infrastructure recommendation or selection system.

Cloud computing models may provide a large variety of services for customers. Infrastructure as a Service (IaaS) is a layer of cloud computing that provides virtualized computing resources over the Internet. The current laaS model delivers hardware services on demand without taking into account application requirements but, as automated decision support systems powered by artificial intelligence are included as part of cloud middleware, laaS is likely to evolve to take into account how customer applications use cloud hardware resources. IaaS is one of three main categories of cloud computing services, alongside Software as a Service (SaaS) and Platform as a Service (PaaS). SaaS is a software licensing and delivery layer or model of cloud computing in which software is licensed on a subscription basis and is centrally hosted. It is sometimes referred to as "on-demand software". PaaS is another layer of cloud computing services that provide a platform allowing customers to develop, run, and manage web applications without the complexity of building and maintaining the infrastructure typically associated with developing and launching an application.

Hybrid cloud infrastructure is a cloud computing environment which uses a mix of on-premises, private cloud and third-party, public cloud services with orchestration between the two platforms. By allowing workloads to move between private and public clouds as computing needs and costs change, hybrid cloud gives businesses greater flexibility and more data deployment options. Cloud brokers help users to choose among the best cloud providers. However, cloud brokering is a difficult task and its quality of service (QoS) depends on many factors, such as finding the required computational resources that guarantee the performance of applications and services (it should be noted that, in the context of the present specification, QoS may be considered as "the acceptable cumulative effect on subscriber satisfaction of all imperfections affecting the service"; other terms with similar meaning to QoS are the quality of experience (QoE) subjective business concept, the required "user perceived performance", the required "degree of satisfaction of the user", and the targeted "number of happy customers"). Cloud brokers base their recommendations on evaluations of the service of public cloud providers, and on users' knowledge. A previously-proposed system employs templates to define infrastructures that are initially defined by users. Once a template is defined, different cloud public providers are evaluated to find the right provider to deploy the requested resources. A performance monitoring tool is used to analyse the system usage, and this information is later used by a recommendation system to update and improve the templates. In such a system, recommendations are based on predefined user requirements to execute an application, but do not take into account how the application uses the cloud infrastructure.

US2013/0198723A1 discloses mapping of High Performance Computing ("HPC") applications to platforms. An HPC application characterization module is provided to determine an HPC application signature to characterize the HPC application. An HPC application mapping module selects a platform from a plurality of platforms to execute the HPC application based on the HPC application signature and a set of benchmarks. An HPC application monitoring module monitors the execution of the HPC application on the selected platform.

It is desirable to provide an improved cloud infrastructure recommendation or selection system.

According to an embodiment of a first aspect there is provided a cloud infrastructure recommendation method to identify at least one preferred cloud infrastructure system, from amongst cloud infrastructure systems provided in at least one private cloud system and at least one public cloud system, to execute a specified computer application. The method comprises providing a register of computer applications for which usage profiles, comprising performance data and energy consumption data, derived from execution of the applications computing systems in private or public cloud systems, are stored. At least some of the performance and/or energy consumption data may be quantitative. The usage profiles may be derived from execution of the applications on physical or virtual computing systems. The method further comprises: determining which applications in the register have usage profiles which are similar to a usage profile of the specified computer application; selecting, from computing systems associated with the applications determined to have similar usage profiles, a first set of computing systems which, on the basis of the performance data and energy consumption data, meet a minimum performance requirement or an energy consumption criterion; selecting a second set of computing systems on the public cloud system which have the most similar resources to the computing systems of the first set; and identifying as the at least one preferred cloud infrastructure system the system which corresponds to the computing system having a lowest predicted energy consumption of those computing systems of the first and second sets that have a predicted performance meeting a desired performance level. Each usage profile of the applications in the register has an associated classification, which is such that usage profiles which are similar have the same classification, and determining similar usage profiles comprises finding usage profiles with same classification as the specified application. Selecting a second set of computing systems comprises selecting the top Ncp computing systems from the public cloud system having the most similar resources to the computing systems in the first set, where Ncp is an integer, and predicting the performance and energy consumption of executing the specified computer application on each computing system in the second set.

Selecting a first set of computing systems may comprise selecting from the computing systems with similar usage profiles a first group comprising the top Np computing systems from those having the best performance and a second group comprising the top N_{E} computing systems from those which are the most energy efficient, where Np and N_{E} are integers.

In embodiments the usage profile of each computer application in the register may further comprise a prediction of the energy consumption and the performance of the specified computer application as a function of the system resources. A prediction algorithm trained on the energy consumption and performance data of virtual systems with similar usage profiles may be used to predict energy consumption and performance.

Embodiments may further comprise, before determining which applications have similar usage profiles, checking if the specified computer application is already registered in the register, and, if not registered, registering the specified computer application in the register and carrying out a profiling process to obtain the usage profile of the specified computer application.

The profiling process may comprise: creating a virtual system template in accordance with hardware requirements of the specified computer application; deploying the virtual system template on the private cloud system; executing the specified computer application on the virtual system; obtaining data indicative of at least the performance of the specified computer application on the virtual system and the energy consumption of the virtual system when executing the specified computer application; and storing that data as the usage profile of the specified computer application.

Embodiments may further comprise classifying the usage profile of the specified computer application such that the usage profile has the same classification as similar usage profiles of applications in the register.

Embodiments may further comprise deploying the application on the preferred cloud infrastructure system.

In embodiments the register may be initially populated with a set of benchmarking applications for which usage profiles have been derived by executing each benchmarking application at least once on the private cloud system.

According to an embodiment of a second aspect there is provided a computer program which, when run on a computer, causes that computer to carry out a method embodying the first aspect.

According to an embodiment of a third aspect there is provided a cloud infrastructure recommendation apparatus to identify at least one preferred cloud infrastructure system, from amongst cloud infrastructure systems provided in at least one private cloud system and at least one public cloud system, to execute a specified computer application. The apparatus comprises a register of computer applications for which usage profiles, comprising performance data and energy consumption data, derived from execution of the applications in private or public cloud systems, are stored. At least some of the performance data or at least some of the energy consumption data may be quantitative. The usage profiles may be derived from execution of the applications on physical or virtual computing systems. The apparatus further comprises: a database to store the usage profiles; and a processor to: determine which applications in the register have usage profiles which are similar to a usage profile of the specified computer application; select, from computing systems associated with the applications determined to have similar usage profiles, a first set of computing systems which, on the basis of the performance data and energy consumption data, meet a minimum performance requirement or an energy consumption criterion; select a second set of computing systems on the public cloud system which have the most similar resources to the computing systems of the first set; and identify as the at least one preferred cloud infrastructure system the system which corresponds to the computing system having the lowest predicted energy consumption of those computing systems of the set that have a predicted performance meeting a desired performance level. Each usage profile of the applications in the register has an associated classification, which is such that usage profiles which are similar have the same classification, and determining similar usage profiles comprises finding usage profiles with same classification as the specified application. Selecting a second set of computing systems comprises selecting the top N_{CP} computing systems from the public cloud system having the most similar resources to the computing systems in the first set, where N_{CP} is an integer, and predicting the performance and energy consumption of executing the specified computer application on each computing system in the second set.

Embodiments may further comprise an application profiler to check, before determining which applications have similar usage profiles, if the specified computer application is already registered in the register, and, if not registered, to: register the specified computer application in the register, and carry out a profiling process to obtain the usage profile of the specified computer application.

According to an embodiment of a fourth aspect there is provided computer apparatus associated with a private cloud system, the computer apparatus comprising: a cloud infrastructure recommendation apparatus to select a preferred computer infrastructure system, from the private cloud system or at least one public cloud system, to execute a specified computer application; and a deployer to deploy the specified computer application on the preferred infrastructure system; the cloud infrastructure recommendation apparatus comprising apparatus embodying the third aspect.

Embodiments provide a method and apparatus for generating at least one recommendation for hybrid cloud users that want to know which private or public cloud resources are the most suitable for their application based on quantitative criteria, including estimated energy consumption. This allows a user to choose a system which minimises or at least reduces the amount of energy required to execute the application. There may be several recommendations, including both private cloud and public cloud resources. The resources may be recommended on the basis of energy consumption and performance estimations using usage profiles for applications previously-executed on computing systems (e.g. virtual machines, containers and/or physical machines) in private or public cloud systems. In the context of this application at least, the term "cloud" is taken to mean any means, available now or in the future, by which computing services or facilities are delivered or made available to computers or other computing devices through the Internet.

Logs containing usage information of applications may be used for training and validation of a classification algorithm that classifies the applications according to how they use a system. Once applications have been registered and classified, the recommendation system has access to a set of data on computing systems for each class that may be used by the recommendation system to filter among those resources that are available on the public cloud providers.

An embodiment may be used by private cloud users or public cloud providers.

Application profiling data obtained in respect of data centre applications may help to clarify how users use private cloud systems. In particular, energy consumption data of the data centre which is stored for different applications and use cases may help data centre users to design new strategies that improve energy efficiency and job scheduling or take advantage of renewable energies. For example, the energy consumption of data centres may be minimized by using public cloud providers for some applications.

The application profiling data may be used to help public cloud providers decide whether to upgrade a data centre to improve performance and/or energy efficiency.

Reference will now be made, by way of example, to the accompanying drawings, in which:
Figure 1A is an overview flowchart of a method embodying the present invention;
Figure 1B is a block diagram of apparatus embodying the present invention;
Figure 2 is a flowchart of an application profiling method;
Figure 3 is a flowchart of an application profiling process using benchmarking applications;
Figure 4 is a diagram for use in explaining automatic set-up of private cloud resources for application profiling;
Figure 5 is a diagram illustrating a relational model of databases of a monitoring system;
Figure 6 is a flowchart of a performance and energy consumption prediction workflow;
Figure 7 is a flowchart of a recommendation method; and
Figure 8 is a block diagram of a computing device embodying the present invention.

Embodiments of the present invention relate to a recommendation system and method to help a hybrid cloud user to choose the most energy efficient resources to run a specified application among those available on public cloud providers and the customer's private cloud. The recommendation system is based on quantitative estimations of energy consumption and application performance.

The invention is defined by the independent claims. Further aspects of the invention are outlined in the dependent claims. When the term embodiment is used for describing unclaimed combinations of features, the term has to be understood as referring to examples useful for understanding the present invention.

Nowadays data centre management software is able to monitor performance and energy consumption parameters of a data centre, and this information may be used to reduce the energy consumption of large data centres that belong to public cloud providers. Monitoring information of data centres may also be used for application profiling to create, for instance, energy-aware scheduling algorithms. In addition, thanks to cloud management software and virtualization technologies, it is possible to profile the performance of systems and applications with different hardware configurations.

In embodiments of the present invention, performance estimations are based on a combination of application profiling and historical data collected from data centre logs. Energy consumption estimations are calculated based on the logs of power consumption of the application that have been obtained from historical data of the data centre. Previously-profiled applications are registered in an application catalogue. If one or more changes have been made in the configuration of an application already stored in the application catalogue, such as new input files or data sets, then the changed application is considered as a new application. Application performance and power consumption is evaluated on different computing systems, i.e. virtual systems (virtual machines (VM) and/or containers) and/or physical machines. A set of application benchmarks may be executed for initial profiling. In this case, applications and systems are monitored during the initial profiling and the results are used to provide the first data for a recommendation system. The recommendation system filters similar public cloud services and estimates the application performance of, and the energy which would be consumed by, running the same application on the public cloud. Finally, the top private and public cloud services are ranked based on maximum performance and minimum energy consumption criteria. The cost of running the service on a private cloud may additionally be estimated based on the power consumption and the associated electricity cost. Additional overheads due to the data centre administration and maintenance may be included in the cost estimation.

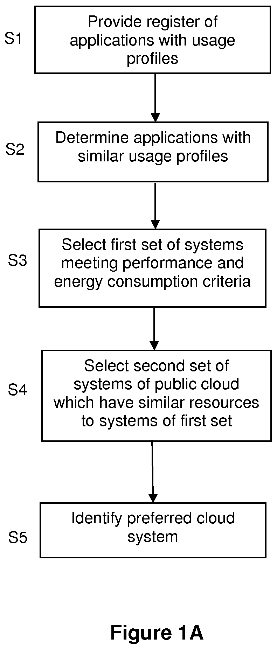

Figure 1A is a flowchart of a cloud infrastructure recommendation method according to an embodiment. The method identifies at least one preferred cloud infrastructure system, from amongst cloud infrastructure systems provided in at least one private cloud system and at least one public cloud system, to execute a specified computer application. The method of the embodiment comprises (step S1) providing a register of computer applications for which usage profiles, comprising quantitative performance data and energy consumption data, derived from execution of the applications on computing systems in private and/or public cloud systems, are stored. Applications in the register which have usage profiles which are similar to a usage profile of the specified computer application are determined (step S2). A set of computing systems, from computing systems associated with the applications determined to have similar usage profiles, which, on the basis of the performance data and energy consumption data, meet a preset minimum performance requirement or a preset energy consumption criterion are selected (step S3). Then a second set of computing systems on the public cloud system which have the most similar resources to the computing systems of the first set are selected (step S4). Finally (step S5), the at least one preferred cloud infrastructure system is identified as the system which corresponds to the computing system having the lowest predicted energy consumption of those computing systems of the set that have a predicted performance meeting a desired performance level.

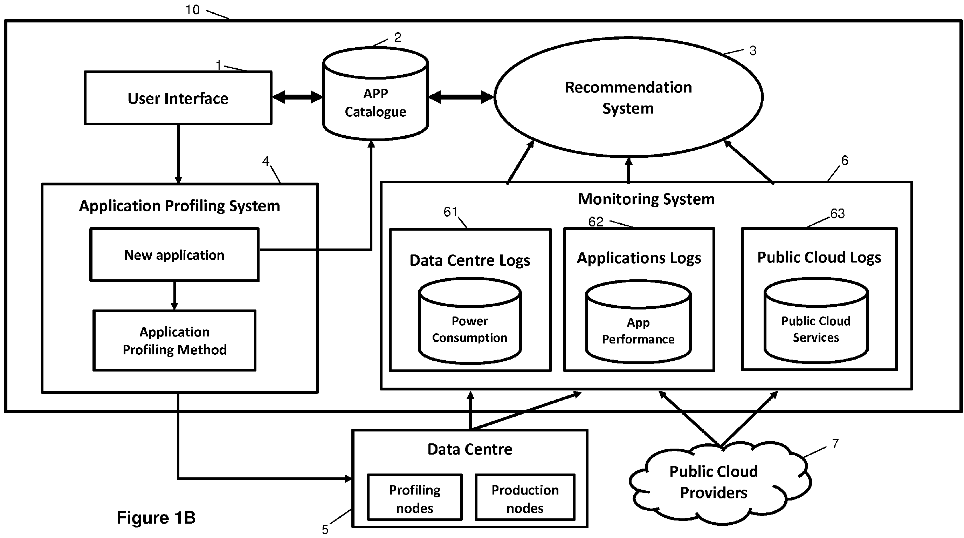

Figure 1B shows an overview block diagram of apparatus 10 embodying the present invention. The apparatus 10 comprises a graphical user interface (GUI) 1, an application catalogue 2 in which applications, their required resources and input parameters (if necessary), have been registered, a recommendation system 3 configured to recommend suitable cloud resources, from available private cloud resources (data centre) 5 and public cloud resources 7, for executing an application, an application profiling system 4 configured to profile the performance of an application, and a monitoring system 6 having databases including a data centre logs database 61, an applications logs database 62 and a public cloud logs database 63.

The GUI 1 is configured to enable a user to choose an application from the application catalogue 2 or to submit a new application. The GUI 1 also allows new data sets for each application and the application set up to be defined. Other parameters such as the minimum hardware set up and the available budget may also be introduced in the GUI 1. Users may also indicate in the GUI the necessary time to solution such as the application execution time or the service availability. This includes execution time of the application, data transfer, visualization, etc. The GUI is also configured to display information indicating the public and/or private cloud systems, with performance and energy consumption estimations, suggested by the recommendation system 3. The GUI 1 communicates with the application profiling system 4 and cloud management software via an application programming interface (API).

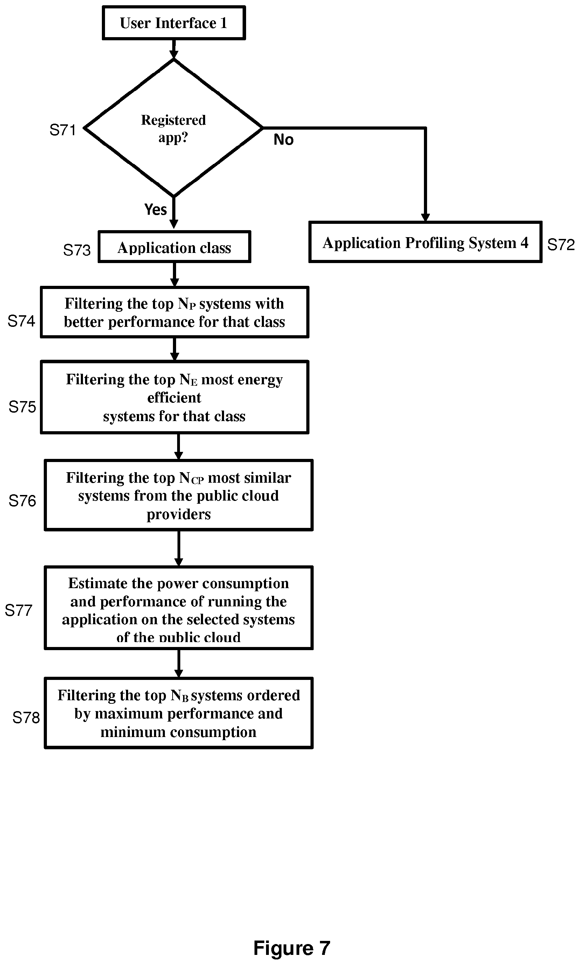

When users have submitted their requests via the GUI 1, as explained later with reference to Figure 7, if the specified application is already registered in the application catalogue 2 the recommendation system 3 uses the information stored in the databases 61, 62 and 63 of the monitoring system 6 to determine at least one private cloud system 5 or public cloud system 7 which is the most suitable for executing he specified application. The data centre logs database 61 and the applications log database 62 respectively store the systems logs (including power consumption data) and performance data for instances of the application executed on the private cloud system 5. Systems logs, performance data and public cloud service data are respectively stored in the data centre logs database 61, the applications logs database 62 and the public cloud logs database 63 for instances of the application executed on a public cloud system 7.

The recommendation system 3 will suggest a private cloud 5 for those applications that are not already registered in the application catalogue 2. In this case the application profiling system 4 is operable to profile the performance of the new application by interacting with the cloud management software, deploying the requested infrastructure, executing the requested application, and monitoring the application and system performance., The application profiling system 4 registers the new application in the application catalogue 2, collects the power consumption data and usage logs from the systems together with the application performance, and stores the collected data in the data centre logs and applications logs databases 61, 62 of the monitoring system 6 for use as the usage profile of the application.

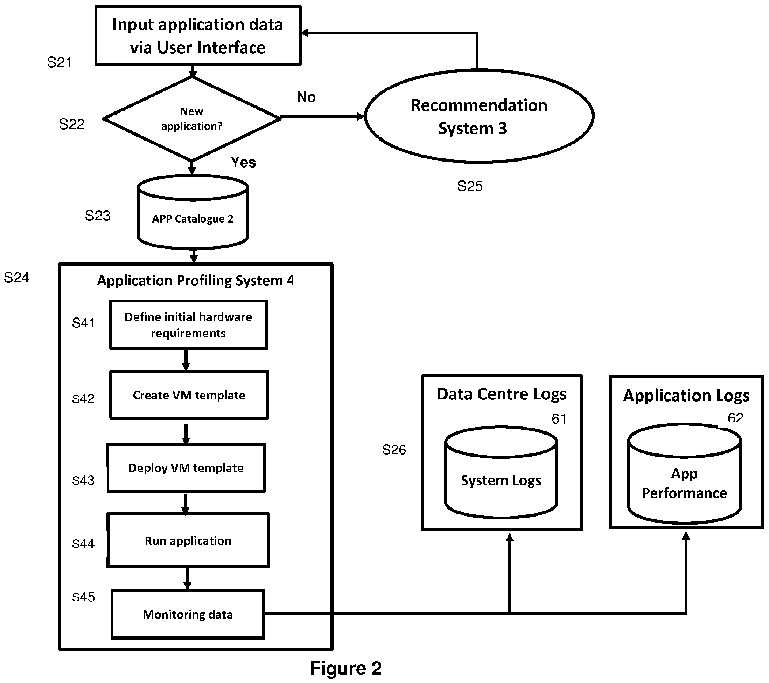

Figure 2 shows a flow chart of a process carried out by the apparatus when a new application is submitted by a user (step S21). If the application is already registered in the application catalogue 2 (No, step S22), the recommendation system 3 is initiated (step S25). If it is determined that the application is a new application (Yes, step S22), the application is registered in the application catalogue 2 (step S23), and the application profiling system carries out an application profiling process (step S24). The application profiling process begins (at step S41) with the user specifying via the GUI 1 the required computational resources for the new application. The application profiling process then creates a virtual system (e.g. virtual machine or container) template based on the input computational resources (step S42), deploys the virtual system template (step S43) and runs the application on the virtual system (step S44). The application profiling system evaluates the application performance on the virtual system corresponding to the requested computational resources and monitors the system usage and power consumption (step S45). The data obtained by the application profiling process is then stored (step S26) as the usage profile of the application in the data centre logs and application logs databases 61 and 62 of the monitoring system 6.

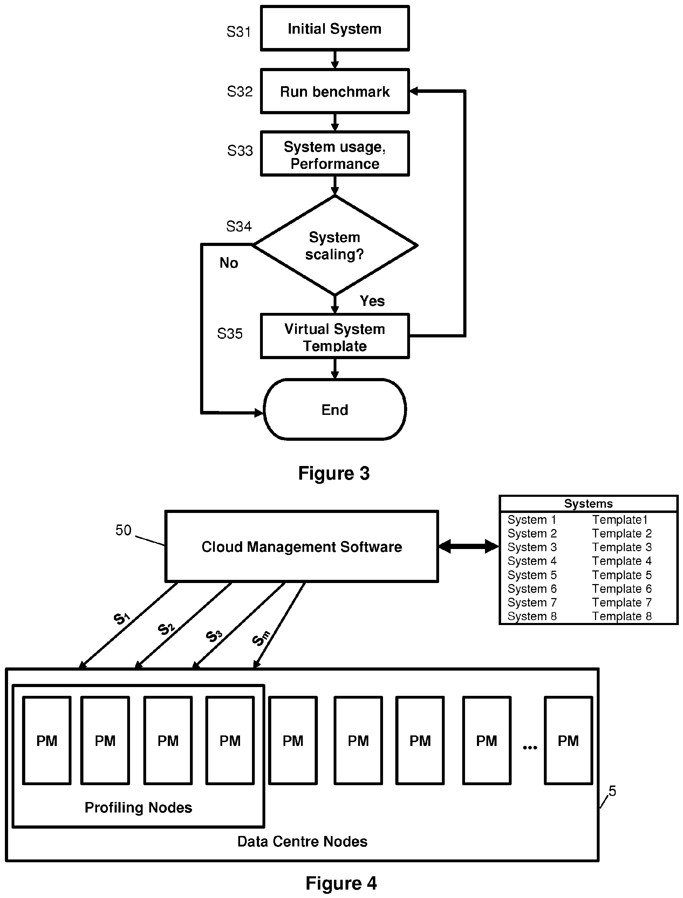

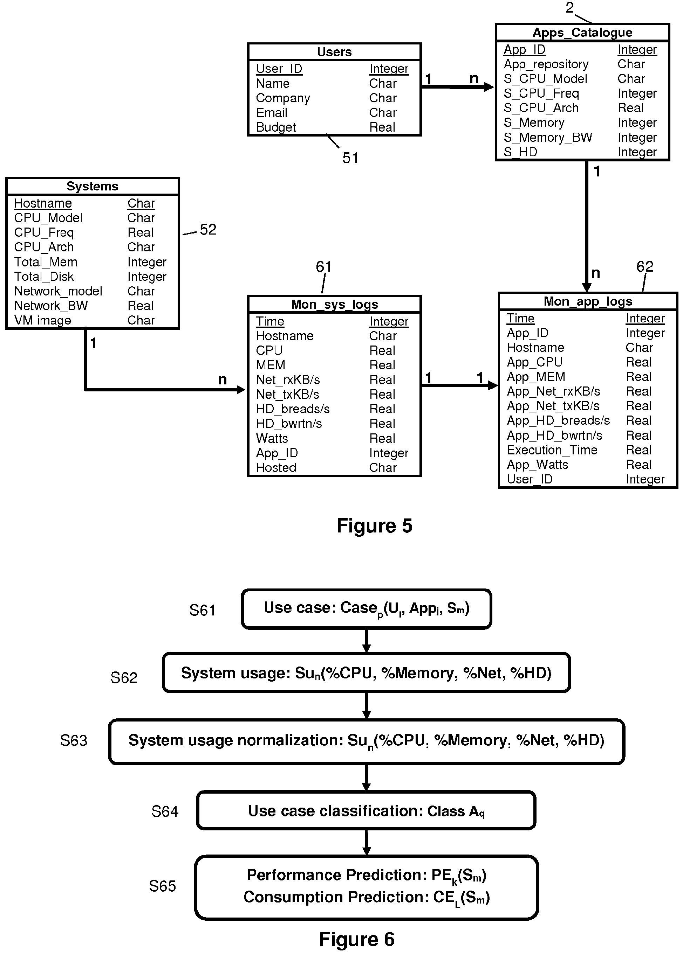

The application catalogue 2 may be initialized with a set of application benchmarks. These will provide data for use in classifying new applications (as will be explained later with reference to Figure 6) when no other data has yet been stored in the application catalogue 2. Figure 3 shows a workflow of a process for generating offline data based on information collected by the application profiling system 4 from running the application benchmarks. Once the initial system is configured according to its application requirements (step S31), each benchmark application is executed (step S32) on a virtual system deployed on the user's private cloud resources and monitoring information related to system usage and application performance obtained is stored (step S33) in the data centre logs and application logs databases 61 and 62 of the monitoring system 6. After storing the monitoring information, the system usage data is evaluated to analyse whether any of the resources of the system are underutilised (step S34). For instance, the system usage may be represented by a four component vector Sᵤ=[%Total CPU, %Memory, %Network bandwidth, %IO bandwidth] that shows which percentage of the total resources have been used by the application. Those resources that are underutilised are scaled down to create a new virtual system template (step S35) which is used for a new execution of the benchmark applications so as to generate as much information as possible about how the application performance and system usage depend on the requested resources.

Figure 4 shows an example of how the application profiling system 2 interacts with the private cloud management software 50 to deploy the system required by the applications. As mentioned above, once the user defines the initial hardware requirements in step S41 of the application profiling process (Figure 2), in step S42 of the process a virtual system template is created with those requirements if an equivalent template does not exist already. The application profiling process then calls the private cloud management software 50 to deploy the requested virtual system on the private cloud 5.

Figure 5 shows an example of the data stored in the data centre logs database 61 and the application performance database 62 of the monitoring system 6, and the relational model of the data stored in the system. Users are registered in a users' database 51 that stores user information in association with the user ID, such as user names, affiliation, contact information, projects that the user is involved in and budget to deploy their services. The application catalogue 2 stores information related to the applications, such as where to find the application (application repository), the recommended hardware requirements and the identifier of the corresponding virtual system template. The available virtual system templates are stored in a systems table 52 that contains the virtual system information such as CPU, memory size, disk size, network connectivity, and associated virtual system image. When an application is deployed on a system, the monitoring information on the execution of the application on the system is stored in the data centre logs database 61. This database 61 collects the system logs of the physical nodes and deployed virtual systems of the private cloud, and also the logs of the physical nodes (in the case of bare-metal deployments) and virtual system instances deployed in a public cloud. Therefore, it is possible to monitor the impact of the virtual system deployment and execution of the applications on the private cloud power consumption. The performance logs of the executed applications are stored in the applications logs database 62. The performance logs of the applications store the usage percentage of the key resources of the system, i.e. the fraction of the system resources that is used by the application. CPU, memory, network and hard disk bandwidth may be considered as key resources of the system. In addition, key performance indicators of the application may also be stored, such as execution time, transactions per second, etc.

Figure 6 shows a workflow of a prediction process carried out by the application profiling system to analyse the data collected for an application during profiling in order to predict the application performance and the energy consumed to run the application on a private cloud as a function of the system resources. Initially, a "use case" (Caseₚ(Uᵢ, Appⱼ, Sₘ)) for the application is defined as a combination of the user (Uᵢ), application (Appⱼ), and requested resources (Sₘ) (step S61). Historical logs related to system usage (Sᵤ(%CPU, %Memory, %Net, %HD)) for that use case are then retrieved (step S62) and normalized (step S63) by the maximum value of the available resources on private and public clouds to generate a normalized vector (Sᵤₙ(%CPU, %Memory, %Net, %HD)) for each use case. A deep learning classification algorithm is used to classify use cases which have a similar usage profile. The normalized vector is used later for training and testing of the classification algorithm. The use case is assigned a classification (A_{q}) according to the similarity of its usage profile to that of other use cases (step S64). For instance, the method described in US2016/0154875A1 may be used to classify the use cases according to the usage profile similarity. After the use case for the application has been classified, the stored performance and power consumption logs of those applications in the application catalogue 2 that belong to the same class as the use case are used (step S65) for training and testing a performance and power consumption prediction algorithm (Performance Prediction: PEₖ(Sₘ); Consumption Prediction: CE_{L}(Sₘ)) in order to obtain predictions of performance and energy consumption for the application. For instance, a linear regression algorithm may be used for performance and power consumption prediction as a function of the compilation resources.

Figure 7 shows a workflow of a process carried out by the recommendation system 3 for requested applications which have already been registered, i.e. applications that have been classified previously by the application profiling system 2. Initially the process checks if the application is already registered (step S71). If not (No, step S71), the application is profiled by the application profiling system 2 (step S72) and the recommendation system 3 recommends the user's private cloud system. If the application is registered (Yes, step S71), the classification of the application, which has been assigned earlier according to its usage profile using the use case classification system, is obtained (step S73). Those computing systems stored in systems table 52 (including any from the public cloud systems) that belong to the same class as the requested application, i.e. which have a similar usage profile, are then identified and ranked (steps S74 and S75) to obtain a first set comprising two computing system groups:
- a top performance group: i.e. the top N_{P} (e.g. ten) computing systems with the best performance.
- a top energy efficient group: i.e. the top N_{E} (e.g. ten) most energy efficient computing systems.
The recommendation system 3 then selects (step S76) from computing systems in the systems table 52 a second set comprising the top N_{CP} (e.g. ten) computing systems from the public cloud providers with the most similar resources to those in the top performance group and in the top energy efficient group (N_{P}, N_{E} and N_{CP} are arbitrarily selected integers and need not be equal to one another). As a result, the recommendation system 3 has a selection of the equivalent resources available on public cloud providers and makes a prediction (step S77) of the application performance and power consumption of running the application on the public cloud systems. Each computing system in the first and second sets has an associated two component vector (PE, CE) that contains indicators specifying the performance estimation (PE) and consumption estimation (CE) of the computing system (PE and CE are estimated for the selected similar computing systems from public cloud systems). This vector is used to identify and sort the private and public cloud computing systems with the best performance and lowest consumption. As mentioned above with reference to Figure 6 a prediction algorithm, based on the collected data from the profiled applications that belong to the same class as the requested application, is used to estimate the energy consumption and performance of private and public cloud resources. Supervised algorithms of machine learning such as linear regression may be used for these predictions. Finally, N_{B} (e.g. ten) of the selected computing systems from private and public cloud providers are identified and sorted (step S78) according to the performance estimation and power consumption estimation. Information relating to electricity price, administration overheads and maintenance overheads of the private cloud may be used, in addition to the energy consumption logs used to estimate the energy used to run the application on the private cloud, to estimate the cost of running the application.

As an example of how an embodiment may be implemented, consider the case of a user that wants to run a message passing interface (MPI) parallel application, where the application is already registered in the application catalogue 2 and consumes a lot of CPU processing power, memory and network bandwidth. The application profiling system 4 will have already stored the usage profile of the application in the monitoring system 6 and identified a group of applications with similar usage profiles of the system. In this example this group of applications has been classified by the use case classification system as the CPU, memory and network intensive class. A set of virtual systems with similar usage profiles of the system, including power consumption and application performance, is therefore available for training and evaluation of the prediction system. The recommendation system 3 is able to choose a set of similar computing systems from different public cloud providers that may be a good choice for this kind of application and, using the monitoring information, predicts the performance and energy consumption when the application is run on this set of computing systems from public cloud providers, taking into account the profiling information of similar applications that belong to the same class. As a result, the recommendation system 3 generates a ranking of the computing systems of public cloud providers suitable for this application together with a two component vector that contains performance and power consumption indicators. The same vector is also created for any other computing systems that belong to the same class. This enables the recommendation system 3 to suggest those computing systems with better performance and lower power consumption. For those computing systems executed on the private cloud, the estimation of the power consumption may be converted into a monetary cost taking into account the current electricity price and additional overheads if they are provided by the users. For public cloud computing systems, the estimation of application performance may be taken into account to estimate the total cost of executing the application taking into account public prices.

An embodiment may be installed as a service on the user side to support a user's decision to choose between private and/or public clouds. Alternatively, an embodiment may also be suitable for public cloud providers and cloud brokers to recommend to users the most suitable cloud provider based on energy consumption and performance estimations. As mentioned above, for a private cloud installation on a user data centre, the application profiling system 4 may automatically execute a set of benchmarking applications on the private cloud. The logs of application performance and system performance from executing the benchmarking applications are stored in the monitoring system 6. This information may be used for training and evaluation of the classification system of registered applications. The benchmarking set of applications preferably contains a variety of applications representative of the different profiles of resource consumption, such as network intensive applications or CPU and memory intensive applications. The application profiling system 4 may also take advantage of the virtualization capabilities of the private cloud and its algorithm may try to test different configurations to profile the application performance and power consumption as a function of the consumed resources. The automatic benchmarking system may not be necessary for public cloud providers because they should be able to collect the required information from a large variety of applications.

Embodiments of the present invention may be implemented in hardware, or as software modules running on one or more processors, or on a combination thereof. That is, those skilled in the art will appreciate that a microprocessor or digital signal processor (DSP) may be used in practice to implement some or all of the functionality described above.

The invention may also be embodied as one or more device or apparatus programs (e.g. computer programs and computer program products) for carrying out part or all of the methods described herein. Such programs embodying the present invention may be stored on computer-readable media, or could, for example, be in the form of one or more signals. Such signals may be data signals downloadable from an Internet website, or provided on a carrier signal, or in any other form.

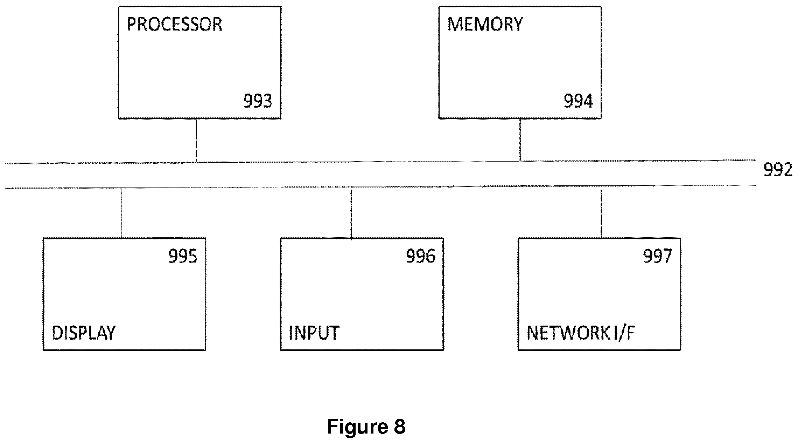

Figure 8 is a block diagram of a computing device, such as a data storage server, which embodies the present invention, and which may be used to implement some or all of the steps of a method embodying the present invention, and perform some or all of the tasks of apparatus of an embodiment. For example, the computing device of Figure 8 may be used to implement all, or only some, of steps S1 to S5 of the method illustrated in Figure 1A, to perform all, or only some, of the tasks of apparatus 10 shown in Figure 1B, to perform all, or only some, of the tasks of GUI 1, application catalogue 2, recommendation system 3, application profiler 4, and/or monitoring system 6, and/or to provide some or all of databases 61, 62 and/or 63, of Figure 1B.

The computing device comprises a processor 993, and memory, 994. Optionally, the computing device also includes a network interface 997 for communication with other such computing devices, for example with other computing devices of invention embodiments.

For example, an embodiment may be composed of a network of such computing devices. Optionally, the computing device also includes one or more input mechanisms such as keyboard and mouse 996, and a display unit such as one or more monitors 995. The components are connectable to one another via a bus 992.

The memory 994, which may provide some or all of databases 61, 62 and/or 63, may include a computer readable medium, which term may refer to a single medium or multiple media (e.g., a centralized or distributed database and/or associated caches and servers) configured to carry computer-executable instructions or have data structures stored thereon. Computer-executable instructions may include, for example, instructions and data accessible by and causing a general purpose computer, special purpose computer, or special purpose processing device (e.g., one or more processors) to perform one or more functions or operations. Thus, the term "computer-readable storage medium" may also include any medium that is capable of storing, encoding or carrying a set of instructions for execution by the machine and that cause the machine to perform any one or more of the methods of the present disclosure. The term "computer-readable storage medium" may accordingly be taken to include, but not be limited to, solid-state memories, optical media and magnetic media. By way of example, and not limitation, such computer-readable media may include non-transitory computer-readable storage media, including Random Access Memory (RAM), Read-Only Memory (ROM), Electrically Erasable Programmable Read-Only Memory (EEPROM), Compact Disc Read-Only Memory (CD-ROM) or other optical disk storage, magnetic disk storage or other magnetic storage devices, flash memory devices (e.g., solid state memory devices).

The processor 993 is configured to control the computing device and execute processing operations, for example executing computer program code stored in the memory 994 to implement the methods described with reference to Figures 1A, 2, 3, 6 and/or 7 and defined in the claims. The memory 994 stores data being read and written by the processor 993. As referred to herein, a processor may include one or more general-purpose processing devices such as a microprocessor, central processing unit, or the like. The processor may include a complex instruction set computing (CISC) microprocessor, reduced instruction set computing (RISC) microprocessor, very long instruction word (VLIW) microprocessor, or a processor implementing other instruction sets or processors implementing a combination of instruction sets. The processor may also include one or more special-purpose processing devices such as an application specific integrated circuit (ASIC), a field programmable gate array (FPGA), a digital signal processor (DSP), network processor, or the like. In one or more embodiments, a processor is configured to execute instructions for performing the operations and steps discussed herein.

The display unit 995 may display a representation of data stored by the computing device and may also display a cursor and dialog boxes and screens enabling interaction between a user and the programs and data stored on the computing device. The input mechanisms 996 may enable a user to input data and instructions to the computing device. The display unit 995 and input mechanisms 996 may form the GUI 1.

The network interface (network I/F) 997 may be connected to a network, such as the Internet, and is connectable to other such computing devices via the network. The network I/F 997 may control data input/output from/to other apparatus via the network. Other peripheral devices such as microphone, speakers, printer, power supply unit, fan, case, scanner, trackerball etc may be included in the computing device.

Methods embodying the present invention may be carried out on a computing device such as that illustrated in Figure 8. Such a computing device need not have every component illustrated in Figure 8, and may be composed of a subset of those components. A method embodying the present invention may be carried out by a single computing device in communication with one or more data storage servers via a network. The computing device may be a data storage itself storing at least a portion of the data.

A method embodying the present invention may be carried out by a plurality of computing devices operating in cooperation with one another. One or more of the plurality of computing devices may be a data storage server storing at least a portion of the data.

The above-described embodiments of the present invention may advantageously be used independently of any other of the embodiments or in any feasible combination with one or more others of the embodiments.

## Claims

1. A method for selecting a cloud infrastructure system comprising:
providing a register of computer applications for which usage profiles, comprising performance data and energy consumption data, derived from execution of the applications on computing systems in private or public cloud systems, are stored;
**characterised by**:
determining which applications in the register have usage profiles which are similar to a usage profile of a specified computer application,
wherein each usage profile of the applications in the register has an associated classification, which is such that usage profiles which are similar have the same classification; and
wherein determining similar usage profiles comprises finding usage profiles with the same classification as the specified computer application;
selecting, from computing systems associated with the applications determined to have similar usage profiles, a first set of computing systems which, on the basis of the performance data and energy consumption data, meet a minimum performance requirement or an energy consumption criterion;
selecting a second set of computing systems on the public cloud system which have the most similar resources to the computing systems of the first set by selecting the top N_{CP} computing systems from the public cloud system having the most similar resources to the computing systems in the first set, where N_{CP} is an integer;
predicting the performance and energy consumption of executing the specified computer application on computing systems in the second set; and
identifying as a preferred cloud infrastructure system the system which corresponds to the computing system having a lowest predicted energy consumption of those computing systems of the first and second sets that have a predicted performance meeting a desired performance level.

2. A method as claimed in claim 1, wherein selecting a first set of computing systems comprises:
selecting from the computing systems with similar usage profiles a first group comprising the top N_{P} computing systems from those having the best performance and a second group comprising the top N_{E} computing systems from those which are the most energy efficient, where N_{P} and N_{E} are integers.

3. A method as claimed in any preceding claim, wherein the usage profile of each computer application in the register further comprises a prediction of the energy consumption and the performance of the specified computer application as a function of the system resources.

4. A method as claimed in claim 3, wherein a prediction algorithm trained on the energy consumption and performance data of virtual systems with similar usage profiles is used to predict energy consumption and performance.

5. A method as claimed in any preceding claim, further comprising, before determining which applications have similar usage profiles, checking if the specified computer application is already registered in the register, and if not registered:
registering the specified computer application in the register; and
carrying out a profiling process to obtain the usage profile of the specified computer application.

6. A method as claimed in claim 5, wherein the profiling process comprises:
creating a virtual system template in accordance with hardware requirements of the specified computer application;
deploying the virtual system template on the private cloud system;
executing the specified computer application on the virtual system;
obtaining data indicative of at least the performance of the specified computer application on the virtual system and the energy consumption of the virtual system when executing the specified computer application; and
storing that data as the usage profile of the specified computer application.

7. A method as claimed in claim 6, further comprising classifying the usage profile of the specified computer application such that the usage profile has the same classification as similar usage profiles of applications in the register.

8. A method as claimed in any preceding claim, further comprising deploying the application on the preferred cloud infrastructure system.

9. A method as claimed in any preceding claim, wherein the register is initially populated with a set of benchmarking applications for which usage profiles have been derived by executing each benchmarking application at least once on the private cloud system.

10. A computer program which, when run on a computer, causes that computer to carry out a method as claimed in any preceding claim.

11. Cloud infrastructure recommendation apparatus (10) to select a cloud infrastructure system comprising:
a register (2) of computer applications for which usage profiles, comprising performance data and energy consumption data, derived from execution of the applications on computing systems in private or public cloud systems (5, 7), are stored;
a database (62) to store the usage profiles; and **characterised by** a processor (3) configured to:
determine which applications in the register (2) have usage profiles which are similar to a usage profile of a specified computer application,
wherein each usage profile of the applications in the register has an associated classification, which is such that usage profiles which are similar have the same classification; and
wherein determining similar usage profiles comprises finding usage profiles with the same classification as the specified application;
select, from computing systems associated with the applications determined to have similar usage profiles, a first set of computing systems which, on the basis of the performance data and energy consumption data, meet a minimum performance requirement or an energy consumption criterion;
select a second set of computing systems on the public cloud system (7) which have the most similar resources to the computing systems of the first set by selecting the top N_{CP} computing systems from the public cloud system (7) having the most similar resources to the computing systems in the first set, where N_{CP} is an integer;
predict the performance and energy consumption of executing the specified computer application on computing systems in the second set; and
identify as a preferred cloud infrastructure system the system which corresponds to the computing system having the lowest predicted energy consumption of those computing systems of the set that have a predicted performance meeting a desired performance level.

12. Apparatus as claimed in claim 11, further comprising an application profiler to check, before determining which applications have similar usage profiles, if the specified computer application is already registered in the register, and if not registered to:
register the specified computer application in the register; and
carry out a profiling process to obtain the usage profile of the specified computer application.

13. Computer apparatus associated with a private cloud system (5), the computer apparatus comprising:
a cloud infrastructure recommendation apparatus (10) to select a preferred computer infrastructure system, from the private cloud system (5) or at least one public cloud system (7), to execute a specified computer application; and
a deployer to deploy the specified computer application on the preferred infrastructure system;
the cloud infrastructure recommendation apparatus comprising apparatus as claimed in claim 11 or 12.

## Patentansprüche

1. Verfahren zum Auswählen eines Cloud-Infrastruktursystems, Folgendes umfassend:
Bereitstellen eines Registers von Computeranwendungen, für welche Verwendungsprofile,
welche Leistungsdaten und Energieverbrauchsdaten umfassen, welche aus einer Ausführung der Anwendungen auf Datenverarbeitungssystemen in privaten oder öffentlichen Cloud-Systemen abgeleitet wurden, gespeichert sind;
**gekennzeichnet durch**:
Bestimmen, welche Anwendungen in dem Register Verwendungsprofile aufweisen, welche einem Verwendungsprofil einer spezifizierten Computeranwendung ähnlich sind,
wobei jedes Verwendungsprofil der Anwendungen in dem Register eine dazugehörige Klassifizierung aufweist, welche derartig ist, dass Verwendungsprofile, welche ähnlich sind, die gleiche Klassifizierung aufweisen; und
wobei Bestimmen ähnlicher Verwendungsprofile Auffinden von Verwendungsprofilen mit der gleichen Klassifizierung wie die spezifizierte Computeranwendung umfasst;
Auswählen, aus Datenverarbeitungssystemen, welchen den Anwendungen zugeordnet sind, über welche bestimmt wurde, dass sie ähnliche Verwendungsprofile aufweisen, eines ersten Satzes Datenverarbeitungssysteme, welche auf der Grundlage der Leistungsdaten und Energieverbrauchsdaten eine minimale Leistungsanforderung oder ein minimales Energieverbrauchskriterium erfüllen;
Auswählen eines zweiten Satzes Datenverarbeitungssysteme auf dem öffentlichen Cloud-System, welches die ähnlichsten Betriebsmittel zu den Datenverarbeitungssystemen des ersten Satzes aufweisen, durch Auswählen der obersten N_{CP} Datenverarbeitungssysteme aus dem öffentlichen Cloud-System, welche die ähnlichsten Betriebsmittel zu den Datenverarbeitungssystemen in dem ersten Satz aufweisen, wobei N_{CP} eine ganze Zahl ist;
Vorhersagen des Leistungsvermögens und des Energieverbrauchs des Ausführens der spezifizierten Computeranwendung auf Datenverarbeitungssystemen in dem zweiten Satz; und
Identifizieren, als ein bevorzugtes Cloud-Infrastruktursystem, des Systems, welches dem Datenverarbeitungssystem mit einem niedrigsten vorhergesagten Energieverbrauch von diesen Datenverarbeitungssystemen des ersten und des zweiten Satzes entspricht, welche ein vorhergesagtes Leistungsvermögen aufweisen, welches eine erwünschte Leistungsstufe erfüllt.

2. Verfahren nach Anspruch 1, wobei Auswählen eines ersten Satzes von Datenverarbeitungssystemen, Folgendes umfasst:
Auswählen aus den Datenverarbeitungssystemen mit ähnlichen Verwendungsprofilen einer ersten Gruppe, welche die obersten Nₚ Datenverarbeitungssysteme von denen mit dem besten Leistungsvermögen umfasst, und einer zweiten Gruppe, welche die obersten N_{E} Datenverarbeitungssysteme von denen umfasst, welche die energieeffizientesten sind, wobei Nₚ und N_{E} ganz Zahlen sind.

3. Verfahren nach einem der vorhergehenden Ansprüche, wobei das Verwendungsprofil jeder Computeranwendung in dem Register weiterhin eine Vorhersage des Energieverbrauchs und des Leistungsvermögens der spezifizierten Computeranwendung als eine Funktion der Systembetriebsmittel umfasst.

4. Verfahren nach Anspruch 3, wobei ein Vorhersagealgorithmus, welcher an den Energieverbrauchs- und Leistungsdaten virtueller Systeme mit ähnlichen Verwendungsprofilen trainiert ist, verwendet wird, um Energieverbrauch und Leistungsvermögen vorherzusagen.

5. Verfahren nach einem der vorhergehenden Ansprüche, weiterhin umfassend, vor Bestimmen, welche Anwendungen ähnliche Verwendungsprofile aufweisen, Prüfen, ob die spezifizierte Computeranwendung bereits in dem Register registriert ist, und, wenn sie nicht registriert ist, Registrieren der spezifizierten Computeranwendung in dem Register; und
Ausführen eines Profilbestimmungsprozesses, um das Verwendungsprofil der spezifizierten Computeranwendung zu ermitteln.

6. Verfahren nach Anspruch 5, wobei der Profilbestimmungsprozess Folgendes umfasst:
Erzeugen einer virtuellen Systemvorlage gemäß Hardware-Anforderungen der spezifizierten Computeranwendung;
Einsetzen der virtuellen Systemvorlage auf dem privaten Cloud-System;
Ausführen der spezifizierten Computeranwendung auf dem virtuellen System;
Ermitteln von Daten, welche für mindestens das Leistungsvermögen der spezifizierten Computeranwendung auf dem virtuellen System und dem Energieverbrauch des virtuellen Systems beim Ausführen der spezifizierten Computeranwendung bezeichnend ist; und
Speichern dieser Daten als das Verwendungsprofil der spezifizierten Computeranwendung.

7. Verfahren nach Anspruch 6, weiterhin umfassend Klassifizieren des Verwendungsprofils der spezifizierten Computeranwendung, so dass das Verwendungsprofil die gleiche Klassifizierung aufweist wie ähnliche Verwendungsprofile von Anwendungen in dem Register.

8. Verfahren nach einem der vorhergehenden Ansprüche, weiterhin umfassend Einsetzen der Anwendung auf dem bevorzugten Cloud-Infrastruktursystem.

9. Verfahren nach einem der vorhergehenden Ansprüche, wobei das Register anfänglich mit einem Satz von Benchmarking-Anwendungen besetzt ist, für welche Verwendungsprofile durch Ausführen jeder Benchmarking-Anwendung mindestens einmal auf dem privaten Cloud-System abgeleitet wurden.

10. Computerprogramm, welches, wenn es auf einem Computer läuft, bewirkt, dass dieser Computer ein Verfahren nach einem der vorhergehenden Ansprüche ausführt.

11. Cloud-Infrastruktur-Empfehlungsvorrichtung (10), um ein Cloud-Infrastruktursystem auszuwählen, Folgendes umfassend:
ein Register (2) von Computeranwendungen, für welche Verwendungsprofile, welche Leistungsdaten und Energieverbrauchsdaten umfassen, welche aus einer Ausführung der Anwendungen auf Datenverarbeitungssystemen in privaten oder öffentlichen Cloud-Systemen (5, 7) abgeleitet wurden, gespeichert sind;
eine Datenbank (62), um die Verwendungsprofile zu speichern; und **gekennzeichnet durch** einen Prozessor (3), welcher konfiguriert ist, um:
zu bestimmen welche Anwendungen in dem Register (2) Verwendungsprofile aufweisen, welche einem Verwendungsprofil einer spezifizierten Computeranwendung ähnlich sind,
wobei jedes Verwendungsprofil der Anwendungen in dem Register eine dazugehörige Klassifizierung aufweist, welche derartig ist, dass Verwendungsprofile, welche ähnlich sind, die gleiche Klassifizierung aufweisen; und
wobei Bestimmen ähnlicher Verwendungsprofile Auffinden von Verwendungsprofilen mit der gleichen Klassifizierung wie die spezifizierte Anwendung umfasst;
aus Datenverarbeitungssystemen, welche den Anwendungen zugeordnet sind, über welche bestimmt wurde, dass sie ähnliche Verwendungsprofile aufweisen, einen ersten Satz Datenverarbeitungssysteme auszuwählen, welche auf der Grundlage der Leistungsdaten und Energieverbrauchsdaten eine minimale Leistungsanforderung oder ein minimales Energieverbrauchskriterium erfüllen;
einen zweiten Satz Datenverarbeitungssysteme auf dem öffentlichen Cloud-System (7), welches die ähnlichsten Betriebsmittel zu den Datenverarbeitungssystemen des ersten Satzes aufweist, durch Auswählen der obersten N_{CP} Datenverarbeitungssysteme aus dem öffentlichen Cloud-System (7) auszuwählen, welche die ähnlichsten Betriebsmittel zu den Datenverarbeitungssystemen in dem ersten Satz aufweisen, wobei N_{CP} eine ganze Zahl ist;
das Leistungsvermögen und den Energieverbrauch des Ausführens der spezifizierten Computeranwendung auf Datenverarbeitungssystemen in dem zweiten Satz vorherzusagen; und
als ein bevorzugtes Cloud-Infrastruktursystem das System zu identifizieren, welches dem Datenverarbeitungssystem mit dem niedrigsten vorhergesagten Energieverbrauch von diesen Datenverarbeitungssystemen des Satzes entspricht, welcher ein vorhergesagtes Leistungsvermögen aufweist, welches eine erwünschte Leistungsstufe erfüllt.

12. Vorrichtung nach Anspruch 11, weiterhin umfassend einen Anwendungsprofilbestimmer, um vor Bestimmen, welche Anwendungen ähnliche Verwendungsprofile aufweisen, zu prüfen, ob die spezifizierte Computeranwendung bereits in dem Register registriert ist, und, wenn sie nicht registriert ist, um:
die spezifizierte Computeranwendung in dem Register zu registrieren; und
einen Profilbestimmungsprozess auszuführen, um das Verwendungsprofil der spezifizierten Computeranwendung zu ermitteln.

13. Computervorrichtung, welche einem privaten Cloud-System (5) zugeordnet ist, die Computervorrichtung Folgendes umfassend:
eine Cloud-Infrastruktur-Empfehlungsvorrichtung (10), um ein bevorzugtes Computerinfrastruktursystem aus dem privaten Cloud-System (5) oder mindestens einem öffentlichen Cloud-System (7) auszuwählen, um eine spezifizierte Computeranwendung auszuführen; und
einen Einsetzer, um die spezifizierte Computeranwendung auf dem bevorzugten Infrastruktursystem einzusetzen;
wobei die Cloud-Infrastruktur-Empfehlungsvorrichtung eine Vorrichtung nach Anspruch 11 oder 12 umfasst.

## Revendications

1. Procédé pour sélectionner un système d'infrastructure de nuage comprenant :
la prévision d'un registre d'applications informatiques pour lesquelles des profils d'utilisation, comprenant des données de performance et des données de consommation d'énergie, déduites de l'exécution des applications sur des systèmes informatiques dans des systèmes de nuage privés ou publics, sont mémorisés ;
**caractérisé par** :
la détermination des applications dans le registre qui ont des profils d'utilisation qui sont similaires à un profil d'utilisation d'une application informatique spécifiée,
dans lequel chaque profil d'utilisation des applications dans le registre a une classification associée, qui est telle que les profils d'utilisation qui sont similaires ont la même classification ; et
dans lequel la détermination de profils d'utilisation similaires comprend la recherche de profils d'utilisation avec la même classification que l'application informatique spécifiée ;
la sélection, parmi les systèmes informatiques associés aux applications déterminées comme ayant des profils d'utilisation similaires, d'un premier ensemble de systèmes informatiques qui, sur la base des données de performance et des données de consommation d'énergie, satisfont à un besoin de performance minimum ou à un critère de consommation d'énergie ;
la sélection d'un deuxième ensemble de systèmes informatiques sur le système de nuage public qui ont les ressources les plus similaires aux systèmes informatiques du premier ensemble en sélectionnant les N_{CP} systèmes informatiques supérieurs dans le système de nuage public ayant les ressources les plus similaires aux systèmes informatiques dans le premier ensemble, où N_{CP} est un entier ;
la prédiction de la performance et de la consommation d'énergie de l'exécution de l'application informatique spécifiée sur les systèmes informatiques dans le deuxième ensemble ; et
l'identification, en tant que système d'infrastructure de nuage préféré, du système qui correspond au système informatique ayant une consommation d'énergie prédite la plus faible de ces systèmes informatiques des premier et deuxième ensembles qui ont une performance prédite satisfaisant à un niveau de performance souhaité.

2. Procédé selon la revendication 1, dans lequel la sélection d'un premier ensemble de systèmes informatiques comprend :
la sélection, parmi les systèmes informatiques avec des profils d'utilisation similaires, d'un premier groupe comprenant les N_{P} systèmes informatiques supérieurs parmi ceux ayant la meilleure performance et d'un deuxième groupe comprenant les N_{E} systèmes informatiques supérieurs parmi ceux qui ont le meilleur rendement énergétique, où N_{P} et N_{E} sont des entiers.

3. Procédé selon l'une quelconque des revendications précédentes, dans lequel le profil d'utilisation de chaque application informatique dans le registre comprend en outre une prédiction de la consommation d'énergie et de la performance de l'application informatique spécifiée en fonction des ressources de système.

4. Procédé selon la revendication 3, dans lequel un algorithme de prédiction appris concernant les données de consommation d'énergie et de performance de systèmes virtuels avec des profils d'utilisation similaires est utilisé pour prédire la consommation d'énergie et la performance.

5. Procédé selon l'une quelconque des revendications précédentes, comprenant en outre, avant la détermination des applications qui ont des profils d'utilisation similaires, la vérification que l'application informatique spécifiée est déjà enregistrée dans le registre, et si elle n'est pas enregistrée :
l'enregistrement de l'application informatique spécifiée dans le registre ; et
l'exécution d'un processus de définition de profil pour obtenir le profil d'utilisation de l'application informatique spécifiée.

6. Procédé selon la revendication 5, dans lequel le processus de définition de profil comprend :
la création d'un modèle de système virtuel conformément aux besoins matériels de l'application informatique spécifiée ;
le déploiement du modèle de système virtuel sur le système de nuage privé ;
l'exécution de l'application informatique spécifiée sur le système virtuel ;
l'obtention de données indicatives au moins de la performance de l'application informatique spécifiée sur le système virtuel et de la consommation d'énergie du système virtuel lors de l'exécution de l'application informatique spécifiée ; et
la mémorisation de ces données en tant que profil d'utilisation de l'application informatique spécifiée.

7. Procédé selon la revendication 6, comprenant en outre la classification du profil d'utilisation de l'application informatique spécifiée de sorte que le profil d'utilisation ait la même classification que les profils d'utilisation similaires des applications dans le registre.

8. Procédé selon l'une quelconque des revendications précédentes, comprenant en outre le déploiement de l'application sur le système d'infrastructure de nuage préféré.

9. Procédé selon l'une quelconque des revendications précédentes, dans lequel le registre est initialement peuplé avec un ensemble d'applications d'analyse comparative pour lesquelles des profils d'utilisation ont été déduits en exécutant chaque application d'analyse comparative au moins une fois sur le système de nuage privé.

10. Programme d'ordinateur qui, lorsqu'il est exécuté sur un ordinateur, amène cet ordinateur à effectuer un procédé selon l'une quelconque des revendications précédentes.

11. Appareil de recommandation d'infrastructure de nuage (10) pour sélectionner un système d'infrastructure de nuage comprenant :
un registre (2) d'applications informatiques pour lesquelles des profils d'utilisation, comprenant des données de performance et des données de consommation d'énergie, déduites de l'exécution des applications sur des systèmes informatiques dans des systèmes de nuage privés ou publics (5, 7), sont mémorisés ;
une base de données (62) pour mémoriser les profils d'utilisation ; et **caractérisé par**
un processeur (3) configuré pour :
déterminer quelles applications dans le registre (2) ont des profils d'utilisation qui sont similaires à un profil d'utilisation d'une application informatique spécifiée,
dans lequel chaque profil d'utilisation des applications dans le registre a une classification associée, qui est telle que les profils d'utilisation qui sont similaires ont la même classification ; et
dans lequel la détermination des profils d'utilisation similaires comprend la recherche de profils d'utilisation avec la même classification que l'application spécifiée ;
sélectionner, parmi les systèmes informatiques associés aux applications déterminées comme ayant des profils d'utilisation similaires, un premier ensemble de systèmes informatiques qui, sur la base des données de performance et des données de consommation d'énergie, satisfont à un besoin de performance minimum ou à un critère de consommation d'énergie ;
sélectionner un deuxième ensemble de systèmes informatiques sur le système de nuage public (7) qui ont les ressources les plus similaires aux systèmes informatiques du premier ensemble en sélectionnant les N_{CP} systèmes informatiques supérieurs dans le système de nuage public (7) ayant les ressources les plus similaires aux systèmes informatiques dans le premier ensemble, où N_{CP} est un entier ;
prédire la performance et la consommation d'énergie de l'exécution de l'application informatique spécifiée sur les systèmes informatiques dans le deuxième ensemble ; et
identifier, en tant que système d'infrastructure de nuage préféré, le système qui correspond au système informatique ayant la consommation d'énergie prédite la plus faible de ces systèmes informatiques de l'ensemble qui ont une performance prédite satisfaisant à un niveau de performance souhaité.

12. Appareil selon la revendication 11, comprenant en outre un dispositif de définition de profil d'application pour vérifier, avant la détermination des applications qui ont des profils d'utilisation similaires, si l'application informatique spécifiée est déjà enregistrée dans le registre, et si elle n'est pas enregistrée :
enregistrer l'application informatique spécifiée dans le registre ; et
effectuer un processus de définition de profil pour obtenir le profil d'utilisation de l'application informatique spécifiée.

13. Appareil informatique associé à un système de nuage privé (5), l'appareil informatique comprenant :
un appareil de recommandation d'infrastructure de nuage (10) pour sélectionner un système d'infrastructure d'ordinateur préféré, dans le système de nuage privé (5) ou au moins dans un système de nuage public (7), pour exécuter une application informatique spécifiée ; et
un dispositif de déploiement pour déployer l'application informatique spécifiée sur le système d'infrastructure préféré ;
l'appareil de recommandation d'infrastructure de nuage comprenant un appareil selon la revendication 11 ou 12.
